# FASCICULE DE BREVET EUROPEEN

(11) **EP 0 641 572 B1**
(45) Date de publication et mention de la délivrance du brevet: **26.04.2000**
(21) Numéro de dépôt: 94401759.9
(22) Date de dépôt: 01.08.1994
(51) Int. Cl.: A61N 1/37

(54) **Circuit de protection pour dispositif électronique implantable**
Schutzschaltkreis für ein einpflanzbares elektronisches Gerät
Protection circuit for implantable electronic device

(30) Priorité: 11.08.1993 FR 9309853
(43) Date de publication de la demande: 08.03.1995
(60) Demande divisionnaire: 99106100.3
(73) Titulaire: ELA MEDICAL (Société anonyme), F-92541 Montrouge Cédex (FR)
(72) Inventeur: Jacobson, Peter, F-67500 Haguenau (FR)
(74) Mandataire: Laget, Jean-Loup

(56) Documents cités:
- EP-A- 0 546 666
- US-A- 4 320 763
- US-A- 4 745 923
- US-A-4 440 172
- US-A-4 595 009
- US-A-5 111 816

## Description

L'invention concerne la protection des dispositifs électroniques implantables comprenant des électrodes utilisées pour détection ou stimulation à faible énergie, vis-à-vis d'impulsions de forte énergie. Elle comprend les stimulateurs cardiaques implantables et les défibrillateurs/stimulateurs cardiaques implantables.

Dans le cas présent, le terme défibrillateur est utilisé pour désigner tout dispositif pour éliminer une tachyarythmie par une énergie électrique dépassant sensiblement l'énergie fournie par les stimulateurs cardiaques implantables.

L'invention concerne la protection des circuits reliés aux électrodes à faible énergie, et la protection des tissus du patient sur le site des électrodes à faible énergie, vis-à-vis des propres impulsions de forte énergie du dispositif lui-même, et des impulsions de forte énergie provenant d'autres dispositifs électroniques médicaux tels que des défibrillateurs externes.

L'invention s'applique également à des dispositifs électroniques ayant besoin d'une protection contre des impulsions de forte énergie.

Les stimulateurs cardiaques implantables assurent généralement une protection vis-à-vis d'impulsions externes de forte énergie par une limitation de la tension entre une paire quelconque d'électrodes. Quand un dispositif externe applique une impulsion de forte énergie à cette paire d'électrodes à travers une faible impédance, des courants de forte intensité passent dans les électrodes et le dispositif limiteur. Ces courants de forte intensité peuvent avoir un effet nuisible sur les tissus du patient dans la région des électrodes.

Le brevet US No 4 320 763 au nom de Money décrit un dispositif limiteur de courant placé en circuit série entre un stimulateur cardiaque et l'extrémité proximale d'une électrode, pour éviter des dommages aux tissus adjacents à l'extrémité distale de l'électrode. Il décrit deux circuits, l'un avec des transistors à effet de champ (FET) et l'autre avec un transistor bipolaire qui limite et ensuite maintient le courant à une valeur fixe d'approximativement 20 mA.

Le brevet US No 4 440 172 au nom de Langer décrit un circuit prévu pour protéger un amplificateur de recueil de l'électrocardiogramme ou ECG vis-à-vis d'impulsions de défibrillation, consistant en une résistance de valeur élevée en série avec l'entrée de l'amplificateur, et en des diodes de limitation appliquées à cette entrée, sur le côté proximal de la résistance. Un circuit de stimulation protégé applique de l'énergie à haute fréquence, par un transformateur d'isolement, à un démodulateur, qui applique de son côté l'impulsion démodulée aux électrodes de stimulation. Une diode à polarisation inverse protège ce circuit pendant la défibrillation.

Le brevet US No 4 595 009 au nom de Leinders décrit un défibrillateur/stimulateur cardiaque qui relie ensemble les électrodes de stimulation et de détection au moment où il fournit une impulsion de défibrillation.

Le brevet US No 4 745 923 au nom de Winstrom décrit un circuit de protection pour un dispositif implantable, relié en série à une sonde d'un stimulateur cardiaque pour le protéger vis-à-vis de fortes tensions et de courants élevés provenant de défibrillateurs et autres sources. Le circuit décrit limite le courant passant dans les deux directions dans une seule sonde (le brevet montre le circuit dans la sonde de retour de la stimulation , ou positive).

Ce circuit utilise une limitation de courant par repli, également fréquemment utilisée dans les alimentations électroniques de puissance du commerce (Section 6,05, Horowitz et Hill, *The Art of Electronics*, Cambridge University Press, 1989). Dans un limiteur de courant par repli, l'impédance reste faible jusqu'à ce que le courant atteigne une première limite préétablie. Quand le courant dépasse cette première limite, l'impédance reste élevée jusqu'à ce que le courant tombe au-dessous d'une seconde limite prédéterminée qui est inférieure à la première.

Le limiteur de Winstrom comporte un parcours de faible impédance entre ses bornes consistant en un transistor à effet de champ (FET) en série avec une résistance de faible valeur. Un transistor bipolaire ouvre le FET quand la tension appliquée à la résistance de faible valeur dépasse le seuil base-émetteur du transistor bipolaire (la première limite préétablie dans le limiteur de courant par repli).

Le brevet US No 4 787 389 au nom de Tarjan décrit un système défibrillateur/stimulateur cardiaque qui ouvre des commutateurs entre le stimulateur cardiaque et ses sondes pendant le temps où il active son défibrillateur.

Le brevet britannique 2 234 908 au nom de Bocchi montre un défibrillateur/stimulateur cardiaque comprenant un commutateur de protection en série avec l'électrode de stimulation, avec un signal de commande pour l'ouvrir pendant la défibrillation et le fermer pendant la stimulation.

Le brevet US No 5 111 816 au nom de Pless montre un défibrillateur/stimulateur cardiaque avec un seul commutateur à transistor à effet de champ métal-oxyde-semiconducteur (MOSFET) à canal N en série avec chaque sonde de stimulation et de détection. Le dispositif ouvre ces commutateurs quand il envoie un choc. Quand ils sont ouverts, ces commutateurs ne permettent pas au courant de passer par les sondes à partir des sources de tension positive par rapport à la masse de stimulation.

Ce brevet montre également un générateur de chocs dont la borne négative de son alimentation de puissance est raccordée à la masse de stimulation de manière qu'il ne produise que des tensions positives par rapport à la masse de stimulation, et donc dans la plage de fonctionnement du circuit de protection.

Il montre en outre, dans le circuit d'application de chocs, une diode en série avec chaque commutateur d'envoi de choc, pour éviter qu'un courant inverse ne passe par les commutateurs du circuit de choc quand des impulsions externes à forte énergie arrivent aux électrodes de choc. Il ajoute des diodes car les commutateurs à semi-conducteur supportent une tension élevée appliquée avec une polarité donnée, mais seulement quelques volts appliqués avec la polarité opposée. Ceci est également valable pour les transistors MOSFET, les transistors bipolaires à jonction, et les transistors bipolaires à porte isolée (IGBT). En faisant en sorte que les commutateurs bloquent le courant dans les deux directions, on évite que le courant passe dans les connexions à l'alimentation de puissance du générateur de chocs pendant des impulsions externes de forte énergie.

Il est donc clair qu'un dispositif électronique implantable tel qu'un défibrillateur/stimulateur cardiaque doit protéger ses sondes de faible énergie, telles que les sondes de stimulation et de détection, vis-à-vis d'impulsions de défibrillation et autres impulsions de forte énergie. L'art antérieur poursuit cet objectif, mais avec des insuffisances:
1. Le brevet Langer US 4.440.172 revendique la protection du circuit de stimulation avec une diode en série avec l'une des électrodes de stimulation. Il est évident que ceci apporte une protection vis-à-vis d'un défibrillateur externe seulement lorsque l'impulsion de sortie du défibrillateur parvient dans une direction préférée sur le circuit de Langer, c'est-à-dire la direction dans laquelle la diode n'est pas conductrice. Le circuit de Langer a également besoin d'un transformateur, qui est difficile à réaliser avec une technologie à circuits intégrés.
2. Le brevet Leinders US 4.595.009 court-circuite les sondes de stimulation et de détection, permettant au courant de défibrillation de passer dans leurs électrodes, avec des effets éventuels sur les tissus du patient qui sont proches de ces électrodes.
3. Les brevets Tarjan US 4.787.389, Bocchi GB 2.234.908 et Pless US 5.111.816 ouvrent les sondes de stimulation et de détection, mais seulement en réponse à un signal de commande provenant de l'implant quand il fournit sa propre impulsion à forte énergie. Ces brevets ne décrivent pas de moyens pour détecter des impulsions de forte énergie provenant de sources externes et pour activer automatiquement les circuits de protection.
4. Le brevet Money US 4.320.763 limite le courant dans les sondes à une valeur fixe, automatiquement. Cependant, la limitation à une valeur fixe exige que le dispositif limiteur doive dissiper une puissance égale au produit de la valeur de la limitation du courant et de la tension appliquée. La puissance appliquée atteint au moins 1000 V à 20 mA, ou 20 W. Le courant dans de tels circuits tend également à osciller autour de la valeur limite, en raison de la forte puissance appliquée. Ces oscillations peuvent avoir un effet néfaste sur le fonctionnement des circuits voisins. En outre, la construction du circuit doit permettre la dissipation de cette puissance.
5. Le brevet Winstrom US 4.745.923 fournit un limiteur de courant par repli automatique, pour surmonter les problèmes d'oscillation et de dissipation posés par une limitation à une valeur constante. Cependant, le circuit de Winstrom n'ouvre le commutateur MOSFET dans un parcours à faible impédance qu'après avoir d'abord commandé un second commutateur à transistor bipolaire, dont le retard peut se traduire par une forte pointe de courant dans le limiteur quand un défibrillateur applique une impulsion à front raide dans les sondes de stimulation et de détection. Le brevet US No 4 823 796 au nom de Benson décrit un défibrillateur externe que le circuit de Winstrom pourrait rencontrer. Benson produit une impulsion trapézoïdale par la décharge par transistor d'un condensateur sans inductance en série. Des mesures montrent que ces circuits peuvent produire des fronts de montée de milliers de volts par microseconde. Tout retard dans le circuit de protection se traduit par une pointe de courant qui peut se coupler à un circuit voisin et l'affecter.
6. Le brevet Winstrom US 4.745.923 décrit seulement un limiteur bidirectionnel qui doit être utilisé dans une sonde unique. L'idée est de limiter le courant dans une sonde d'une paire de sondes quand un défibrillateur applique l'énergie selon l'une ou l'autre polarité. Dans des défibrillateurs/stimulateurs cardiaques à deux chambres, avec deux paires de sondes, le circuit de Winstrom aurait besoin d'être appliqué à trois des quatre conducteurs. Ceci se traduit par un nombre de composants plus élevé que dans le cas d'un limiteur unidirectionnel dans chaque sonde.
7. Le brevet Pless US 5.111.816 décrit un limiteur de courant unidirectionnel en série avec chaque sonde de stimulation et de détection d'un défibrillateur/stimulateur cardiaque, et un circuit de choc qui bloque les impulsions externes de forte énergie. Pless raccorde un côté du générateur de chocs à la masse de la stimulation, de manière que toutes les impulsions de défibrillation que le défibrillateur/stimulateur cardiaque génère soient positives par rapport à la masse de la stimulation, et le limiteur de courant unidirectionnel les bloque dans les sondes de stimulation et de détection. Cependant, le fait de relier le générateur de chocs à la masse exige des tensions de commande positives pour le générateur de chocs, et des tensions de commande négatives pour la stimulation et la détection, ce qui augmente la complexité du circuit.

Le but de la présente invention est donc de fournir une protection améliorée pour les sondes de stimulation et de détection à faible énergie de dispositifs médicaux électroniques, l'amélioration consistant en:
1. La protection vis-à-vis d'impulsions externes de forte énergie de l'une ou l'autre polarité.
2. La limitation du courant passant par les sondes de stimulation et de détection au lieu de la limitation de la tension entre elles, pour protéger les tissus cardiaques dans la région des électrodes de stimulation et de détection.
3. L'activation automatique du limiteur, pour fournir une protection vis-à-vis d'impulsions externes de forte énergie, de même que des impulsions de forte énergie fournies par le dispositif lui-même.
4. Une limitation du courant par repli pour réduire la dissipation de puissance et éliminer les oscillations dans le limiteur.
5. L'activation du limiteur de courant par repli par un unique commutateur à transistor pour fournir une réponse rapide à des impulsions de défibrillation externes à front raide.
6. L'utilisation d'un unique limiteur de courant unidirectionnel dans chaque sonde de stimulation et de détection.
7. Dans une application à un défibrillateur/stimulateur cardiaque implantable, le raccordement du générateur de chocs à une alimentation négative par rapport à la masse de la stimulation, permettant des tensions de commande négatives pour les chocs et la stimulation.

Dans son mode de réalisation préféré, l'invention prévoit un limiteur automatique unidirectionnel de courant par repli dans chaque sonde de stimulation et de détection, qui limite les courants pénétrant dans les sondes. En présence d'une impulsion externe de forte énergie, seul un courant limité peut pénétrer dans chaque sonde de stimulation et de détection, et ainsi seul un courant limité peut passer dans l'une quelconque des électrodes de stimulation et de détection.

Chaque limiteur automatique de courant par repli limite rapidement le courant à une première valeur au moyen d'un unique commutateur à transistor, et un peu plus tard ouvre entièrement ce premier commutateur avec un second commutateur jusqu'à ce que le courant tombe au-dessous d'une seconde valeur plus basse.

Selon un autre aspect de l'invention, quand elle est appliquée à un défibrillateur/stimulateur cardiaque, le circuit générateur de chocs fournit des impulsions positives par rapport à une alimentation négative en basse tension. En outre, les diodes en série avec les commutateurs du générateur de chocs empêchent le courant de passer dans le circuit du générateur de chocs pendant que des impulsions externes de forte énergie sont appliquées aux électrodes de choc.

Les impulsions internes de forte énergie que le générateur de chocs rend positives par rapport à l'alimentation négative en basse tension, ne peuvent passer par les sondes de stimulation que sous forme d'un courant limité dès que leur tension dépasse l'alimentation en basse tension. Comme les impulsions externes de forte énergie ne peuvent pas faire passer le courant dans les électrodes de choc ou dans l'alimentation de puissance, elles ne peuvent donc amener le courant à passer que dans les électrodes de stimulation et de détection, ce que l'invention limite comme expliqué ci-dessus.

L'invention a pour objet un appareil médical comprenant un dispositif de protection du circuit de contrôle et du tissu adjacent aux électrodes, ledit appareil médical étant implanté pour stimulation à faible énergie et détection d'un tissu, ayant des électrodes en contact avec le tissu, un circuit de contrôle pour contrôler la stimulation à faible énergie et la détection, et des sondes reliant les électrodes au circuit de contrôle, le dispositif de protection étant caractérisé en ce qu'il comporte : un unique circuit automatique, unidirectionnel, de limitation du courant, en série avec chaque sonde à faible énergie le circuit de limitation ayant une entrée non protégée du côté du tissu et une sortie protégée du côté du circuit de contrôle.

Selon l'invention :
- chaque circuit de limitation de courant est un limiteur de courant par repli, dans lequel l'impédance reste faible jusqu'à ce que le courant dépasse une première limite préétablie, et reste élevée jusqu'à ce que le courant tombe au dessous d'une seconde limite prédéterminée inférieure à la première ;
- chaque circuit de limitation de courant comporte:
   . une source ayant une tension de polarisation,
   . un dispositif de limitation ayant une chute de tension entre l'entrée et la sortie, comportant un parcours de basse impédance à résistance et transistor, qui présente une première condition de circuit fermé ne limitant pas le courant entre l'entrée et la sortie quand le transistor est passant à l'état normal, une deuxième condition de circuit fermé limitant le courant lorsque la tension aux bornes de la résistance dépasse le seuil du transistor, et une troisième condition de circuit ouvert lorsque le transistor est bloqué, le dispositif de limitation étant sensible à la tension de polarisation et passant automatiquement de la première condition à la deuxième condition; et
   . un moyen de commutation ayant un état inactif et un état actif, l'état actif maintenant le dispositif de limitation dans la troisième condition lorsque le dispositif de limitation est passé automatiquement de la première condition à la deuxième, et la chute de tension entre l'entrée et la sortie du dispositif de limitation dépasse une valeur prédéterminée.
- le dispositif de limitation comprend:
   . un transistor a effet de champ (FET) ayant une source, un drain et une gâchette, le drain étant relié à l'entrée non protégée, le circuit de passage du courant entre le drain et la source du FET étant fermé dans la première condition et ouvert dans la troisème condition;
   . un moyen de détection du courant reliant la source du FET à la sortie protégée ; et
   . un moyen de polarisation pour relier la tension de polarisation à la gâchette du FET, de sorte que la tension entre gâchette et source du FET soit au-dessus de la tension de seuil du FET lorsque le FET est fermé.
- le moyen de détection du courant est constitué par une résistance faible, de préférence de l'ordre de 39 ohms.
- le moyen de commutation comporte en outre :
   . un moyen de monitorage de la chute de tension entre l'entrée et la sortie du dispositif de limitation ;
   . un moyen de changement pour faire passer le moyen de commutation de son état inactif à son état actif lorsque la chute de tension détectée excède une limite prédéterminée ; et
   . un moyen pour mettre le dispositif de limitation dans la troisième condition lorsque le moyen de commutation est dans son état actif.
- le moyen de monitorage est constitué par un diviseur résistif de tension dont le point milieu est relié au moyen de commutation.
- le moyen de commutation et le moyen de changement sont constitués par un transistor bipolaire ayant une base, un émetteur et un collecteur, dans lequel la base du transistor est reliée au moyen de monitorage, le collecteur est relié à la tension de polarisation, et l'émetteur est relié à la sortie protégée.
- le moyen de changement abaisse effectivement la tension de polarisation appliquée au dispositif de limitation.
- l'appareil médical est un dispositif de contrôle cardiaque, de préférence un stimulateur cardiaque.
- l'appareil médical comporte en outre, un moyen générateur de choc pour délivrer un courant de choc au tissu, dans lequel le courant de choc arrivant aux sondes à faible énergie est appliqué selon une polarité à laquelle réagissent lesdits circuits unidirectionnels de limitation de courant.
- l'appareil médical a un potentiel de masse de stimulation et le moyen générateur de choc comporte une alimentation en puissance de choc ayant une tension d'alimentation reliée à une tension de référence qui est voisine dudit potentiel de masse de stimulation.
- la tension de référence est négative et la tension d'alimentation en puissance de choc est positive par rapport à la tension de référence.
- le moyen générateur de choc comporte une paire de sondes utilisables pour délivrer un courant de choc au tissu, une pluralité de commutateurs pour relier sélectivement la tension d'alimentation en puissance de choc à la tension de référence et un moyen de contrôle desdits commutateurs pour délivrer un courant de choc d'une sonde à l'autre avec une polarité choisie, et un moyen de diode en série avec chaque commutateur de choc pour rendre ledit commutateur de choc bidirectionnellement bloquant.
- l'appareil médical est un dispositif de contrôle cardiaque, de préférence un défibrillateur/stimulateur de cardioversion.
- les dispositifs de limitation de courant et le moyen générateur de choc sont construits sous forme d'un circuit intégré hybride.
- le moyen générateur de chocs comporte :
   . une source de tension élevée ;
   . une source de tension de polarisation ;
   . un premier et un deuxième commutateurs montés en série entre les sources de tension élevée et de tension de polarisation ,
   . un troisième et un quatrième commutateurs montés en série entre les sources de tension élevée et de tension de polarisation, chacun des premiers, deuxième, troisième et quatrième commutateurs ayant une condition de circuit ouvert et une condition de circuit fermé ;
   . une première sonde de choc reliée entre les premier et deuxième commutateurs et une deuxième sonde de choc reliée entre les troisième et quatrième commutateurs ;
   . un moyen pour contrôler les premier, deuxième, troisième et quatrième commutateurs pour délivrer un courant de choc entre la source de tension élevée et la source de tension de polarisation à travers les première et deuxième sondes, ledit courant de choc ayant une première polarité à travers les première et deuxième sondes lorsque les premier et quatrième commutateurs sont fermés et les deuxième et troisième commutateurs sont ouverts, et la polarité inverse lorsque les troisième et deuxième commutateurs sont fermés, et les premier et quatrième commutateurs sont ouverts ;
   . une pluralité de diodes telles qu'une diode est branchée chaque fois entre : le premier commutateur et la source de tension élevée, le troisième commutateur et la source de tension élevée, le deuxième commutateur est la première sonde de choc, et le quatrième commutateur et la deuxième sonde de choc, rendant ainsi le générateur de choc bidirectionnellement bloquant.

Selon l'invention :
- la construction du générateur de choc est caractérisée par une structure de circuit intégré hybride ;
- chacun des premier, deuxième, troisième et quatrième commutateurs est constitué par un transistor bipolaire à gâchette isolée ayant une gâchette, un émetteur et un collecteur, avec le collecteur sur le côté haute tension du transistor, et la pluralité de diodes reliées respectivement aux transistors avec la cathode de la diode reliée au collecteur du transistor.
- chacun des transistors bipolaires et des diodes présente une tension de rupture élevée, de préférence de l'ordre de 1 000 volts.

La figure 1 est une vue d'ensemble de l'invention appliquée à un défibrillateur/stimulateur cardiaque.
La figure 2 est un diagramme schématique du générateur de chocs.
La figure 3 est un diagramme schématique d'un limiteur unidirectionnel de courant par repli.

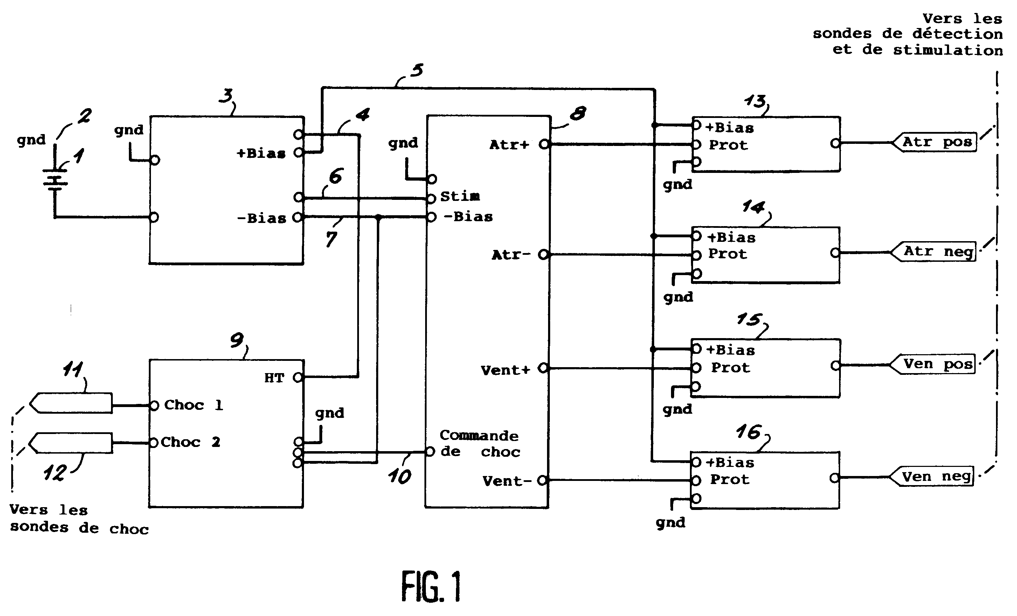

La figure 1 est une vue d'ensemble de l'invention appliquée à un défibrillateur/stimulateur cardiaque, avec des circuits de stimulation et de détection auriculaires et ventriculaires, et avec des chocs multiphasés. La pile 1 alimente le dispositif. La masse 2 sert de tension de référence (0 volts) pour tous les signaux du dispositif, sauf indication contraire. Une alimentation de puissance 3 convertit la tension de la pile en d'autres tensions pour alimenter le reste du dispositif:
* haute tension en 4 pour le générateur de chocs, approximativement 0,75 KV. Le dispositif est couplé à la polarisation négative et non à la masse.
* polarisation positive en 5, pour les limiteurs, d'approximativement 9,0V.
* tension de stimulation en 6, d'approximativement -6,0 V.
* polarisation négative en 7 pour les circuits basse tension et le générateur de chocs, d'approximativement -9,0 V.

En se référant toujours à la figure 1, les circuits basse tension 8 fournissent la stimulation, la détection et la commande. Ils fonctionnent entre la masse 2 et la polarisation négative 7. Comme la valeur de la tension de stimulation en 6 est comprise entre ces deux tensions d'alimentation, ces circuits basse tension peuvent alors générer et mesurer les signaux de stimulation et de détection directement sans décalage de niveau. Quand les circuits basse tension détectent des signaux auriculaires et/ou ventriculaires qui nécessitent un choc, ils le signalent au générateur de chocs 9 par le bus de commande de choc 10.

Toujours à la figure 1, le générateur de chocs 9 envoie des chocs à haute tension entre les sondes de chocs 11 et 12, quand les circuits basse tension le déclenchent par l'intermédiaire du bus de commande de chocs 10. L'énergie de choc provient de la haute tension 4 avec référence à la polarisation négative 7.

Finalement, à la figure 1, les limiteurs 13 à 16 protègent les circuits basse tension 8, et le tissu adjacent aux électrodes de stimulation et de détection reliées aux sondes 17 à 20, vis-à-vis des chocs appliqués par le générateur de chocs 9 ou par un défibrillateur externe.

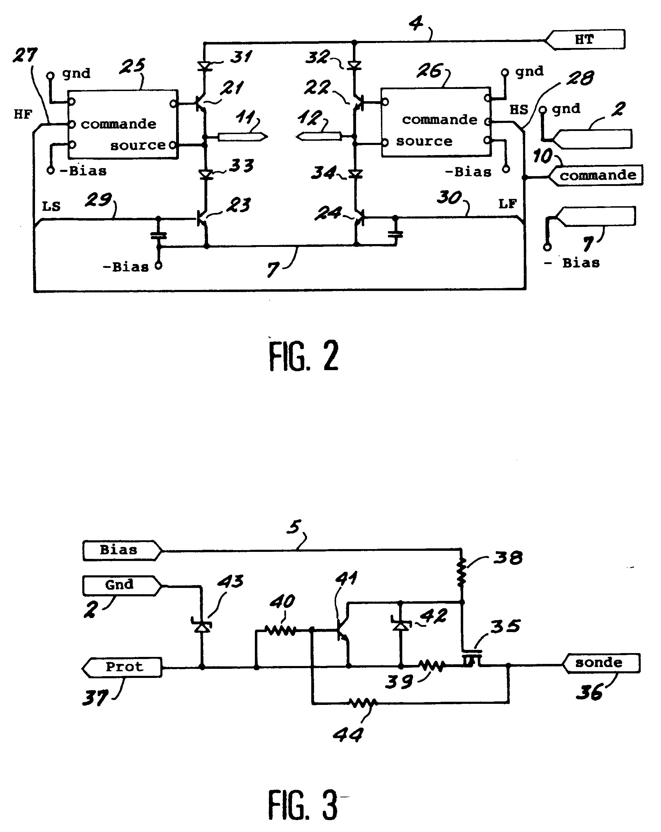

En se référant maintenant à la figure 2, le générateur de chocs représenté à la figure 1 en 9 fonctionne avec quatre commutateurs 21 à 24 configurés sous forme d'un "pont en H" utilisé habituellement pour envoyer à travers une charge un courant alternatif, à partir d'une alimentation en courant continu. Les commutateurs 21 à 24 sont de préférence des transistors bipolaires à gâchette isolée (IGBT). Les isolateurs 25 et 26 ferment les commutateurs du côté supérieur 21 et 22 respectivement en réponse aux signaux de commande HF en 27 et HS en 28, respectivement. Les signaux de commande LS en 29 et LF en 30 ferment les commutateurs 23 et 24 du côté inférieur, respectivement. Comme les sources des commutateurs 23 et 24 sont reliées à la polarisation négative, les signaux de commande LF et LS fonctionnent entre la polarisation négative et la masse, sans convertisseur de tension. Le circuit n'a besoin d'isolateurs que pour les commutateurs 21, 22 du côté supérieur, du fait que les sources des commutateurs 21 et 22 atteignent une tension élevée pendant un choc.

Le circuit de la figure 2 génère alors un choc monophasé ou multiphasé. Quand les circuits basse tension 8 envoient des signaux de commande HF en 27 et LF en 30, ceci provoque la fermeture des commutateurs 21 et 24 et le courant circule depuis l'alimentation haute tension 4, à travers le commutateur 21, la sonde de choc 1 en 11, la sonde de choc 2 en 12, et puis par le commutateur 24 jusqu'à l'alimentation à polarisation négative 7. Comme expliqué ci-dessus, l'alimentation de puissance 3 fournit la tension élevée 4 en référence à la polarisation négative 7. Alternativement, quand les circuits basse tension détectent HS en 28 et LS en 29, le circuit envoie le courant en direction opposée entre les sondes de choc.

Les diodes 31 à 34 empêchent le courant inverse de passer dans les commutateurs 21 à 24 pendant l'application d'une impulsion de tension, provenant d'une source externe, aux électrodes de choc 11 et 12. La tension impulsionnelle externe doit dépasser la somme des tensions de rupture des diodes et des transistors pour que le courant passe entre les électrodes de choc, et elle doit dépasser soit la tension de rupture de la diode soit celle du transistor pour que le courant passe ailleurs (soit vers la polarisation négative, soit vers la masse).

Comme le circuit de la figure 2 relie le générateur de chocs à l'alimentation de polarisation négative, la tension à l'une ou l'autre des électrodes de choc 11 ou 12, pendant un choc généré par le dispositif, doit tomber dans la gamme comprise entre la tension de polarisation négative et la tension élevée. En outre, tout choc externe qui ne dépasse pas la tension de rupture de la diode ou du transistor dans le circuit n'amène pas le courant à passer par l'une ou l'autre électrode de choc, et n'amène donc pas le courant à passer dans l'une ou l'autre des alimentations de puissance: masse, polarisation négative ou haute tension.

En se référant maintenant à la figure 3, les circuits limiteurs, représentés à la figure 1 en 13 à 16, comprennent chacun un commutateur à transistor haute tension 35, de préférence un MOSFET à canal N. Ces transistors existent dans le commerce avec petite surface de silicium, avec des seuils d'approximativement 5 Volts et des résistances en conduction qui sont typiquement inférieures à 10 Ohms pour une tension entre gâchette et source de 9 Volts; par exemple, les types industriels IRFBG20 ou BUK456/1000B.

A la figure 3, les tensions à la borne 36 de la sonde et à la borne 37 de protection du limiteur, pendant le fonctionnement normal et en l'absence d'impulsions internes ou externes de haute énergie, sont comprises entre celle de l'alimentation de polarisation négative et quelques centaines de millivolts au-dessus de la masse.

Alors que les tensions en 36 et 37 restent dans cette plage de fonctionnement normal, la tension de polarisation positive maintient le transistor 35 à l'état passant, par l'intermédiaire de la résistance 38. Comme le transistor 35 ne tire pas de courant de gâchette à l'exception du courant de fuite, l'alimentation de polarisation positive n'a besoin que d'un faible courant. Comme l'alimentation doit générer l'alimentation de polarisation positive en inversant la tension de la pile, cette faible demande de courant permet d'utiliser un petit inverseur capacitif avec peu de composants, et éventuellement intégrable. Si par contre l'architecture de l'invention plaçait la pile au-dessus de la masse et inversait la tension de la pile pour obtenir la stimulation, ceci exigerait une capacité de courant beaucoup plus élevée dans l'inverseur (notamment pour une stimulation rapide pour une réversion d'arythmie), éliminant la possibilité d'une intégration.

Le circuit de la figure 3 comprend une résistance de faible valeur 39 en série avec la source du transistor 35. La résistance constitue avec le transistor 35 un parcours de basse impédance entre la sonde 36 et la connexion protégée 37 quand le transistor 35 est passant à l'état normal dans une première condition de circuit fermé ne limitant pas le courant entre l'entrée 36 et la sortie 37. Si le courant à travers la résistance 39 augmente de manière que la tension aux bornes de cette résistance dépasse le seuil du transistor 35, ceci limite le courant dans le transistor 35 à sa tension de seuil divisée par la valeur de la résistance 39, soit typiquement 0,15 A.Le transistor 35 est alors dans une deuxième condition de limitation du courant.

Quand le transistor 35 limite ainsi le courant, la tension à la borne 36 de la sonde monte jusqu'à ce que la tension base-émetteur du transistor 41, fixée par un diviseur de tension consistant en des résistances 44 et 40, dépasse le seuil de ce transistor 41. Ceci rend le transistor 41 passant, limitant la tension gâchette-source du transistor 35 à zéro. Ceci bloque le transistor 35 et il ne dissipe plus de puissance.Il est alors dans une troisième contition de circuit ouvert, et entre l'entrée 36 et la sortie 37, se trouve la résistance élevée et fixe 44-40. Le circuit reste dans cet état d'impédance élevée jusqu'à ce que la tension à ses bornes tombe à un point où le transistor 41 est bloqué, permettant à la résistance 38 de rendre à nouveau le transistor 35 passant, rétablissant le parcours à faible impédance.

Les diodes Zener 42 et 43 de la figure 3 protègent la jonction gâchette-source du transistor 35, et le circuit basse tension 8 de la figure 1, respectivement.

Bien que l'invention ait été décrite en référence à un mode de réalisation particulier, on comprendra que ce mode de réalisation soit simplement illustratif de l'application des principes de l'invention. De nombreuses autres modifications peuvent être faites et d'autres agencements peuvent être envisagés sans s'écarter des revendications.

## Revendications

1. Appareil médical comprenant un dispositif de protection du circuit de contrôle et du tissu adjacent aux électrodes, ledit appareil médical étant implanté pour stimulation à faible énergie et détection d'un tissu, ayant des électrodes en contact avec le tissu, un circuit de contrôle pour contrôler la stimulation à faible énergie et la détection, et des sondes reliant les électrodes au circuit de contrôle, le dispositif de protection étant caractérisé en ce qu'il comporte : un unique circuit automatique, unidirectionnel, de limitation du courant, en série avec chaque sonde à faible énergie, le circuit de limitation ayant une entrée non protégée du côté du tissu et une sortie protégée du côté du circuit de contrôle.

2. Appareil selon la revendication 1 caractérisé en ce que chaque circuit de limitation de courant (13-16) est un limiteur de courant par repli, dans lequel l'impédance reste faible jusqu'à ce que le courant dépasse une première limite préétablie, et reste élevée jusqu'à ce que le courant tombe au dessous d'une seconde limite prédéterminée inférieure à la première.

3. Appareil selon la revendication 1, caractérisé en ce que chaque circuit de limitation de courant (13-16) comporte :
- une source ayant une tension de polarisation (5);
- un dispositif de limitation (35, 39, 44, 40) ayant une chute de tension entre l'entrée (36) et la sortie (37), comportant un parcours de basse impédance à résistance (39) et transistor (35), qui présente une première condition de circuit fermé ne limitant pas le courant entre l'entrée (36) et la sortie (37) quand le transistor (35) est passant à l'état normal, une deuxième condition de circuit fermé limitant le courant lorsque la tension aux bornes de la résistance (39) dépasse le seuil du transistor (35), et une troisième condition de circuit ouvert lorsque le transistor (35) est bloqué, le dispositif de limitation étant sensible à la tension de polarisation (5) et passant automatiquement de la première condition à la deuxième condition; et
- un moyen de commutation (41) ayant un état inactif et un état actif, l'état actif maintenant le dispositif de limitation dans la troisième condition lorsque
. le dispositif de limitation est passé automatiquement de la première condition à la deuxième, et
. la chute de tension entre l'entrée et la sortie du dispositif de limitation dépasse une valeur prédéterminée.

4. Appareil selon la revendication 3, caractérisé en ce que le dispositif de limitation comprend:
- un transistor (35) a effet de champ (FET) ayant une source, un drain et une gâchette, le drain étant reliée à l'entrée non protégée (36), le circuit de passage du courant entre le drain et la source du FET étant fermé dans la première condition et ouvert dans la troisième condition ;
- un moyen de détection (39) du courant reliant la source du FET (35) à la sortie protégée (37) ; et
- un moyen de polarisation (38) pour relier la tension de polarisation (5) à la gâchette du FET (35), de sorte que la tension entre gâchette et source du FET soit au-dessus de la tension de seuil du FET lorsque le FET est fermé.

5. Appareil selon la revendication 4, caractérisé en ce que le moyen de détection du courant est constitué par une résistance faible (39), de préférence de l'ordre de 39 ohms.

6. Appareil selon l'une des revendications 3 à 5, caractérisé en ce que le moyen de commutation (41) comporte en outre :
- un moyen (40, 44) de monitorage de la chute de tension entre l'entrée et la sortie du dispositif de limitation ;
- un moyen de changement pour faire passer le moyen de commutation de son état inactif à son état actif lorsque la chute de tension détectée excède une limite prédéterminée ; et
- un moyen pour mettre le dispositif de limitation dans la troisième condition lorsque le moyen de commutation est dans son état actif.

7. Appareil selon la revendication 6, caractérisé en ce que le moyen de monitorage est constitué par un diviseur résistif de tension (44, 40) dont le point milieu est relié au moyen de commutation.

8. Appareil selon l'une des revendications 6 et 7, caractérisé en ce que le moyen de commutation et le moyen de changement sont constitués par un transistor bipolaire (41) ayant une base, un émetteur et un collecteur, dans lequel la base du transistor (41) est reliée au moyen de monitorage, le collecteur est relié à la tension de polarisation (5), et l'émetteur est relié à la sortie protégée (37).

9. Appareil selon l'une des revendications 6 à 8, caractérisé en ce que le moyen de changement abaisse effectivement la tension de polarisation appliquée au dispositif de limitation.

10. Appareil selon l'une des revendications 1 à 9, caractérisé en ce que l'appareil médical est un dispositif de contrôle cardiaque, de préférence un stimulateur cardiaque.

11. Appareil selon l'une des revendications 1 à 10, caractérisé en ce que l'appareil médical comporte en outre, un moyen générateur de choc (9) pour délivrer un courant de choc au tissu, dans lequel le courant de choc arrivant aux sondes à faible énergie est appliqué selon une polarité à laquelle réagissent lesdits circuits (13-16) unidirectionnels de limitation de courant.

12. Appareil selon la revendication 11, caractérisé en ce que l'appareil médical a un potentiel de masse de stimulation et en ce que le moyen générateur de choc (9) comporte une alimentation en puissance de choc ayant une tension d'alimentation reliée à une tension de référence (7) qui est voisine dudit potentiel de masse de stimulation (2).

13. Appareil selon l'une des revendications 11 et 12, caractérisé en ce que la tension de référence (7) est négative et la tension d'alimentation en puissance de choc est positive par rapport à la tension de référence.

14. Appareil selon l'une des revendications 11 à 13, caractérisé en ce que le moyen générateur de choc (9) comporte une paire de sondes (11, 12) utilisables pour délivrer un courant de choc au tissu, une pluralité de commutateurs (21-24) pour relier sélectivement la tension d'alimentation en puissance de choc (4) à la tension de référence (7) et un moyen de contrôle desdits commutateurs pour délivrer un courant de choc d'une sonde à l'autre avec une polarité choisie, et un moyen de diode en série avec chaque commutateur de choc pour rendre ledit commutateur de choc bidirectionnellement bloquant.

15. Appareil selon la revendication 14, caractérisé en ce que l'appareil médical est un dispositif de contrôle cardiaque, de préférence un défibrillateur/stimulateur de cardioversion.

16. Appareil selon l'une des revendications 11 à 15, caractérisé en ce que les dispositifs de limitation de courant (13-16) et le moyen générateur de choc (9) sont construits sous forme d'un circuit intégré hybride.

17. Appareil selon la revendication 11, caractérisé en ce que ledit moyen générateur de chocs (9) comporte :
- une source de tension élevée (4) ;
- une source de tension de polarisation (7) ;
- un premier (21) et un deuxième (23) commutateurs montés en série entre les sources de tension élevée (4) et de tension de polarisation (7) ,
- un troisième (22) et un quatrième (24) commutateurs montés en série entre les sources de tension élevée (4) et de tension de polarisation (7), chacun des premiers (21), deuxième (23), troisième (22) et quatrième (24) commutateurs ayant une condition de circuit ouvert et une condition de circuit fermé ;
- une première sonde de choc (11) reliée entre les premier (21) et deuxième (23) commutateurs et une deuxième sonde de choc (12) reliée entre les troisième (22) et quatrième (24) commutateurs ;
- un moyen pour contrôler les premier, deuxième, troisième et quatrième commutateurs pour délivrer un courant de choc entre la source de tension élevée (4) et la source de tension de polarisation (7) à travers les première (11) et deuxième (12) sondes, ledit courant de choc ayant une première polarité à travers les première et deuxième sondes lorsque les premier (21) et quatrième (24) commutateurs sont fermés et les deuxième (23) et troisième (22) commutateurs sont ouverts, et la polarité inverse lorsque les troisième (22) et deuxième (23) commutateurs sont fermés, et les premier (21) et quatrième (24) commutateurs sont ouverts ;
- une pluralité de diodes (31-34) telles qu'une diode est branchée chaque fois entre : le premier commutateur (21) et la source de tension élevée (4), le troisième commutateur (22) et la source de tension élevée (4), le deuxième commutateur (23) est la première sonde de choc (11), et le quatrième commutateur (24) et la deuxième sonde de choc (12), rendant ainsi le générateur de choc bidirectionnellement bloquant.

18. Appareil selon la revendication 17 dans lequel la construction du générateur de choc est caractérisée par une structure de circuit intégré hybride.

19. Appareil selon la revendication 17, caractérisé en ce que chacun des premier (21), deuxième (23), troisième (22) et quatrième (24) commutateurs est constitué par un transistor bipolaire à gâchette isolée ayant une gâchette, un émetteur et un collecteur, avec le collecteur sur le côté haute tension du transistor, et la pluralité de diodes (31-34) reliées respectivement aux transistors (21-24) avec la cathode de la diode reliée au collecteur du transistor.

20. Appareil selon la revendication 19, caractérisé en ce que chacun des transistors bipolaires (21-24) et des diodes (31-34) présente une tension de rupture élevée, de préférence de l'ordre de 1 000 volts.

## Claims

1. A medical apparatus comprising a device for protecting the control circuit and the tissue adjacent to the electrodes, said medical apparatus being implanted for low-energy stimulation and detection of a tissue, having electrodes in contact with the tissue, a control circuit for controlling the low-energy stimulation and the detection, and probes connecting the electrodes to the control circuit, the protection device being characterised in that it comprises: a single automatic, unidirectional current limitation circuit, in series with each low-energy probe, the limitation circuit having a non-protected input on the tissue side and a protected output on the control circuit side.

2. An apparatus according to Claim 1, characterised in that each current limitation circuit (13-16) is a fallback current limiter, in which the impedance remains low until the current exceeds a first preestablished limit, and remains high until the current falls below a second predetermined limit, lower than the first.

3. An apparatus according to Claim 1, characterised in that each current limitation circuit (13-16) comprises:
- a source having a bias voltage (5);
- a limitation device (35, 39, 44, 40) having a potential drop between the input (36) and the output (37), comprising a low-impedance run with a resistor (39) and a transistor (35), which has a first condition of a closed circuit which does not limit the current between the input (36) and the output (37) when the transistor (35) is conductive in the normal state, a second condition of a closed circuit which limits the current when the voltage at the terminals of the resistor (39) exceeds the threshold of the transistor (35), and a third condition of an open circuit when the transistor (35) is blocked, the limitation device being sensitive to the bias voltage (5) and passing automatically from the first condition to the second condition; and
- a switching means (41) having an inactive state and an active state, the active state keeping the limitation device in the third condition when
- the limitation device has passed automatically from the first condition to the second, and
- the potential drop between the input and the output of the limitation device exceeds a predetermined value.

4. An apparatus according to Claim 3, characterised in that the limitation device comprises:
- a field-effect transistor (FET) (35) having a source, a drain and a gate, the drain being connected to the non-protected input (36), the circuit for passing the current between the drain and the source of the FET being closed in the first condition and open in the third condition;
- a detection means (39) for the current connecting the source of the FET (35) to the protected output (37); and
- a polarisation means (38) for connecting the bias voltage (5) to the gate of the FET (35), such that the voltage between the gate and source of the FET is above the threshold voltage of the FET when the FET is closed.

5. An apparatus according to Claim 4, characterised in that the means for detecting the current is constituted by a weak resistor (39), preferably of the order of 39 ohm.

6. An apparatus according to one of Claims 3 to 5, characterised in that the switching means (41) further comprises:
- a means (40, 44) for monitoring the potential drop between the input and the output of the limitation device;
- a changing means for causing the switching means to pass from its inactive state to its active state when the potential drop detected exceeds a predetermined limit; and
- a means for placing the limitation device in the third condition when the switching means is in its active state.

7. An apparatus according to Claim 6, characterised in that the monitoring means is constituted by a resistive voltage divider (44, 40), the centre point of which is connected to the switching means.

8. An apparatus according to one of Claims 6 and 7, characterised in that the switching means and the changing means are constituted by a bipolar transistor (41) having a base, an emitter and a collector, in which the base of the transistor (41) is connected to the monitoring means, the collector is connected to the bias voltage (5) and the emitter is connected to the protected output (37).

9. An apparatus according to one of Claims 6 to 8, characterised in that the changing means effectively reduces the bias voltage applied to the limitation device.

10. An apparatus according to one of Claims 1 to 9, characterised in that the medical apparatus is a cardiac control device, preferably a cardiac pacemaker.

11. An apparatus according to one of Claims 1 to 10, characterised in that the medical apparatus furthermore comprises an impulse-generating means (9) for delivering an impulse current to the tissue, in which the impulse current arriving at the low-energy probes is applied in a polarity to which said unidirectional current limitation circuits (13-16) react.

12. An apparatus according to Claim 11, characterised in that the medical apparatus has a stimulation mass potential and in that the impulse-generating means (9) comprises an impulse power supply having a supply voltage connected to a reference voltage (7) which is close to said stimulation mass potential (2).

13. An apparatus according to one of Claims 11 and 12, characterised in that the reference voltage (7) is negative and the impulse power supply voltage is positive compared with the reference voltage.

14. An apparatus according to one of Claims 11 to 13, characterised in that the impulse-generating means (9) comprises a pair of probes (11, 12) which can be used to deliver an impulse current to the tissue, a plurality of commutators (21-24) for selectively connecting the impulse power supply voltage (4) to the reference voltage (7) and a control means for said commutators for delivering an impulse current from one probe to the other with a selected polarity, and a diode means in series with each impulse commutator to make said impulse commutator bidirectionally blocking.

15. An apparatus according to Claim 14, characterised in that the medical apparatus is a cardiac control device, preferably a cardioversion defibrillator/pacemaker.

16. An apparatus according to one of Claims 11 to 15, characterised in that the current limitation devices (13-16) and the impulse-generating means (9) are constructed in the form of a hybrid integrated circuit.

17. An apparatus according to Claim 11, characterised in that said impulse-generating means (9) comprises:
- a high-voltage source (4);
- a bias voltage source (7);
- a first (21) and a second (23) commutator mounted in series between the high-voltage source (4) and the bias voltage source (7);
- a third (22) and a fourth (24) commutator mounted in series between the high-voltage source (4) and the bias voltage source (7), each of the first (21), second (23), third (22) and fourth (24) commutators having a condition of an open circuit and a condition of a closed circuit;
- a first impulse probe (11) connected between the first (21) and second (23) commutators and a second impulse probe (12) connected between the third (22) and fourth (24) commutators;
- a means for controlling the first, second, third and fourth commutators to deliver an impulse current between the high-voltage source (4) and the bias voltage source (7) via the first (11) and second (12) probes, said impulse current having a first polarity across the first and second probes when the first (21) and fourth (24) commutators are closed and the second (23) and third commutators (22) are open, and the inverse polarity when the third (22) and second (23) commutators are closed and the first (21) and fourth (24) commutators are open;
- a plurality of diodes (31-34) such that one diode is connected each time between: the first commutator (21) and the high-voltage source (4), the third commutator (22) and the high-voltage source (4), the second commutator (23) and the first impulse probe (11), and the fourth commutator (24) and the second impulse probe (12), thus making the impulse generator bidirectionally blocking.

18. An apparatus according to Claim 17, in which the design of the impulse generator is characterised by a hybrid integrated circuit structure.

19. An apparatus according to Claim 17, characterised in that each of the first (21), second (23), third (22) and fourth (24) commutators is formed by a bipolar transistor with isolated gate, having a gate, an emitter and a collector, with the collector on the high-voltage side of the transistor, and the plurality of diodes (31-34) connected respectively to the transistors (21-24) with the cathode of the diode connected to the collector of the transistor.

20. An apparatus according to Claim 19, characterised in that each of the bipolar transistors (21-24) and the diodes (31-34) has a high interrupting voltage, preferably of the order of 1,000 volts.

## Patentansprüche

1. Medizinisches Gerät mit einer Schutzvorrichtung für eine Steuerungsschaltung und ein an Elektroden angrenzendes Gewebe, wobei das medizinische Gerät zur Stimulation mit niedriger Energie und zur Erfassung eines Gewebes implantiert ist und in Kontakt mit dem Gewebe stehende Elektroden, eine Steuerungsschaltung zur Steuerung der Stimulation mit niedriger Energie und der Erfassung sowie die Elektroden mit der Steuerungsschaltung verbindende Sonden aufweist, wobei die Schutzvorrichtung dadurch gekennzeichnet ist, daß sie eine einzelne automatische Schaltung zur unidirektionalen Strombegrenzung aufweist, die in Reihe zu jeder Sonde mit niedriger Energie geschaltet ist, wobei die Begrenzungsschaltung einen ungeschützten Eingangsanschluß auf Gewebeseite und einen geschützten Ausgangsanschluß auf Seiten der Steuerungsschaltung aufweist.

2. Gerät nach Anspruch 1, dadurch gekennzeichnet, daß jede Schaltung zur Strombegrenzung (13-16) ein Strom-Kippbegrenzer ist, in dem die Impedanz unter einem Wert verbleibt, bei dem der Strom eine erste vorbestimmte Grenze überschreitet, und über einen Wert verbleibt, bei dem der Strom unter eine zweite vorbestimmte Grenze fällt, die kleiner ist als die erste Grenze.

3. Gerät nach Anspruch 1, dadurch gekennzeichnet, daß, jede Schaltung zur Strombegrenzung (13-16) aufweist:
- eine Vorspannungsquelle (5),
- eine Begrenzungsvorrichtung (35, 39, 44, 40) mit einem Spannungsabfall zwischen dem Eingangsanschluß (36) und dem Ausgangsanschluß (37), die eine Niedrigimpedanzstrecke aus einem Widerstand (39) und einem Transistor (35) aufweist, die einen ersten Zustand einer geschlossenen Schaltung, bei dem der Strom zwischen dem Eingangsanschluß (36) und dem Ausgangsanschluß (37) nicht begrenzt ist, wenn der Transistor (35) in den normalen Zustand übergeht, einen zweiten Zustand einer geschlossenen Schaltung, bei dem der Strom begrenzt wird, wenn die Spannung an den Anschlüssen des Widerstands (39) den Schwellenwert des Transistors (35) überschreitet, und einen dritten Zustand einer offenen Schaltung aufweist, wenn der Transistor (35) gesperrt ist, wobei die Begrenzungsvorrichtung empfindlich gegenüber der Vorspannung (5) ist und automatisch von dem ersten Zustand in den zweiten Zustand übergeht, und
- eine Umschalteinrichtung (41) mit einem inaktiven Zustand und einem aktiven Zustand, wobei die Begrenzungsvorrichtung bei dem aktiven Zustand in dem dritten Zustand gehalten wird, wenn
• die Begrenzungsvorrichtung automatisch von der ersten zu der zweiten Zustand übergegangen ist und
• der Spannungsabfall zwischen dem Eingangsanschluß und dem Ausgangsanschluß der Begrenzungsvorrichtung einen vorbestimmten Wert überschreitet.

4. Gerät nach Anspruch 3, dadurch gekennzeichnet, daß die Begrenzungsvorrichtung aufweist:
- einen Feldeffekt-(FET-)Transistor (35) mit einer Source-Elektrode, einer Drain-Elektrode und einer Gate-Elektrode, wobei die Drain-Elektrode mit dem ungeschützten Eingangsanschluß (36) verbunden ist und der Stromdurchgangskreis zwischen der Drain-Elektrode und der Source-Elektrode des FET in dem ersten Zustand geschlossen ist und in dem dritten Zustand offen ist,
- eine Einrichtung (39) zur Stromerfassung, die die Source-Elektrode des FET (35) mit dem geschützten Ausgangsanschluß (37) verbindet und
- eine Vorspannungseinrichtung (38) zur Verbindung der Vorspannung (5) mit der Gate-Elektrode des FET (35), so daß die Spannung zwischen der Gate-Elektrode und der Source-Elektrode des FET oberhalb der Schwellenspannung des FET liegt, wenn der FET geschlossen ist.

5. Gerät nach Anspruch 4, dadurch gekennzeichnet, daß die Einrichtung zur Stromerfassung aus einem niedrigen Widerstand (39) besteht, vorzugsweise in der Größenordnung von 39 Ohm.

6. Gerät nach zumindest einem der Ansprüche 3 bis 5, dadurch gekennzeichnet, daß die Umschalteinrichtung (41) aufweist:
- eine Einrichtung (40, 44) zur Überwachung des Spannungsabfalls zwischen dem Eingangsanschluß und dem Ausgangsanschluß der Begrenzungsvorrichtung,
- eine Änderungseinrichtung zur Überführung der Umschalteinrichtung von ihrem inaktiven Zustand in ihren aktiven Zustand, wenn der erfaßte Spannungsabfall eine vorbestimmte Grenze überschreitet, und
- eine Einrichtung zum Einstellen der Begrenzungsvorrichtung in den dritten Zustand, wenn sich die Umschalteinrichtung in ihrem aktiven Zustand befindet.

7. Gerät nach Anspruch 6, dadurch gekennzeichnet, daß die Überwachungseinrichtung aus einem ohmschen Spannungsteiler (44, 40) besteht, dessen Mittelpunkt mit der Umschalteinrichtung verbunden ist.

8. Gerät nach zumindest einem der Ansprüche 6 und 7, dadurch gekennzeichnet, daß die Umschalteinrichtung und die Änderungseinrichtung aus einem Bipolartransistor (41) mit einer Basis-Elektrode, einer Emitter-Elektrode und einer Kollektor-Elektrode bestehen, wobei die Basis-Elektrode des Transistors (41) mit der Überwachungseinrichtung verbunden ist, die Kollektor-Elektrode mit der Vorspannung (5) verbunden ist und die Emitter-Elektrode mit dem geschützten Ausgangsanschluß (37) verbunden ist.

9. Gerät nach zumindest einem der Ansprüche 6 bis 8, dadurch gekennzeichnet, daß die Änderungseinrichtung die an die Begrenzungsvorrichtung angelegte Vorspannung wirksam verringert.

10. Gerät nach zumindest einem der Ansprüche 1 bis 9, dadurch gekennzeichnet, daß das medizinische Gerät ein Gerät zur Herzregelung ist, vorzugsweise ein Herzstimulator.

11. Gerät nach zumindest einem der Ansprüche 1 bis 10, dadurch gekennzeichnet, daß das medizinische Gerät eine Schockgeneratoreinrichtung (9) zur Abgabe eines Schockstroms an das Gewebe aufweist, wobei der in die Sonden mit niedriger Energie fließende Schockstrom entsprechend einer Polarität angelegt ist, bei der die unidirektionalen Strombegrenzungsschaltungen (13-16) reagieren.

12. Gerät nach Anspruch 11, dadurch gekennzeichnet, daß das medizinische Gerät ein Stimulations-Massepotential aufweist und die Schockgeneratoreinrichtung (9) eine Schockenergiezufuhr umfaßt, die eine mit einer Referenzspannung (7) verbundene Versorgungsspannung aufweist, die in der Nähe des Stimulations-Massepotential liegt.

13. Gerät nach zumindest einem der Ansprüche 11 und 12, dadurch gekennzeichnet, daß die Referenzspannung (7) negativ ist und die Schockenergie-Versorgungsspannung hinsichtlich der Referenzspannung positiv ist.

14. Gerät nach zumindest einem der Ansprüche 11 bis 13, dadurch gekennzeichnet, daß die Schockgeneratoreinrichtung (9) ein Paar Sonden (11, 12), die zur Abgabe eines Schockstroms an das Gewebe verwendbar sind, eine Vielzahl von Umschaltelementen (21-24) zum selektiven Verbinden der Schockenergie-Versorgungsspannung (4) mit der Referenzspannung (7), eine Einheit zur Steuerung der Umschaltelemente zur Abgabe eines Schockstroms von der einen Sonde zu der anderen Sonde mit einer gewählten Polarität und ein in Reihe mit jedem Schock-Umschaltelement geschalteten Diodenelement zur bidirektionalen Sperrung des Schock-Umschaltelements aufweist.

15. Gerät nach Anspruch 14, dadurch gekennzeichnet, daß das medizinische Gerät ein Herzregelungsgerät ist, vorzugsweise ein Defibrillator/Kardioversionsstimulator.

16. Gerät nach zumindest einem der Ansprüche 11 bis 15, dadurch gekennzeichnet, daß die Strombegrenzungsvorrichtungen (13-16) und die Schockgeneratoreinrichtung (9) in Form einer integrierten Hybrid-Schaltung ausgebildet sind.

17. Gerät nach Anspruch 11, dadurch gekennzeichnet, daß die Schockgeneratoreinrichtung (9) aufweist:
- eine Hochspannungsquelle (4),
- eine Vorspannungsquelle (7),
- ein erstes (21) und ein zweites (23) Umschaltelement, die in Reihe geschaltet zwischen der Hochspannungsquelle (4) und der Vorspannungsquelle (7) angebracht sind,
- ein drittes (23) und ein viertes (24) Umschaltelement, die in Reihe geschaltet zwischen der Hochspannungsquelle (4) und der Vorspannungsquelle (7) angebracht sind, wobei jedes der ersten (21), zweiten (23), dritten (22) und vierten (24) Umschaltelemente einen offenen Schaltungszustand und einen geschlossenen Schaltungszustand aufweist,
- eine erste Schocksonde (11), die zwischen dem ersten (21) und dem zweiten (23) Umschaltelement angeschlossen ist, und eine zweite Schocksonde (12), die zwischen dem dritten (22) und dem vierten (24) Umschaltelement angeschlossen ist,
- eine Einheit zur Steuerung des ersten, zweiten, dritten und vierten Umschaltelements zur Abgabe eines Schockstroms zwischen der Hochspannungsquelle (4) und der Vorspannungsquelle (7) über die erste (11) und zweite (12) Sonde, wobei der Schockstrom eine erste Polarität über der ersten und zweiten Sonde, wenn das erste (21) und das vierte (24) Umschaltelement geschlossen sind und das zweite (23) und das dritte (22) Umschaltelement offen sind, sowie die umgekehrte Polarität aufweist, wenn das dritte (22) und das zweite (23) Umschaltelement geschlossen sind und das erste (21) und das vierte (24) Umschaltelement offen sind,
- eine Vielzahl von Dioden (31-34) derart, daß jedesmal eine Diode zwischen dem ersten Umschaltelement (21) und der Hochspannungsquelle (4), dem dritten Umschaltelement (22) und der Hochspannungsquelle, dem zweiten Umschaltelement (23) und der ersten Schocksonde (11) sowie dem vierten Umschaltelement (24) und der zweiten Schocksonde (12) angeschlossen ist, wobei auf diese Weise die Schockgeneratoreinrichtung bidirektional gesperrt bleibt.

18. Gerät nach Anspruch 17, wobei der Aufbau der Schockgeneratoreinrichtung durch einen integrierten Hybrid-Schaltungsaufbau gekennzeichnet ist.

19. Gerät nach Anspruch 17, dadurch gekennzeichnet, daß jedes der ersten (21), zweiten (23), dritten (22) und vierten (24) Umschaltelemente aus einem Bipolartransistor mit isolierter Gate-Elektrode, die eine Gate-Elektrode, eine Emitter-Elektrode und eine Kollektor-Elektrode aufweist, mit der Kollektor-Elektrode auf der Hochspannungsseite des Transistors besteht, und die Vielzahl der Dioden (31-34) jeweils mit den Transistoren (21-24) verbunden ist, wobei die Kathoden-Elektrode der Diode mit der Kollektor-Elektrode des Transistors verbunden ist.

20. Gerät nach Anspruch 19, dadurch gekennzeichnet, daß jeder Bipolartransistor (21-24) und jede Diode (31-34) eine hohe Durchschlagsspannung aufweisen, vorzugsweise in der Größenordnung von 1000 Volt.
